# NEUE EUROPÄISCHE PATENTSCHRIFT

(11) **EP 0 528 305 B2**
(45) Veröffentlichungstag und Bekanntmachung des Hinweises auf die Entscheidung über den Einspruch: **17.10.2001**
(45) Hinweis auf die Patenterteilung: 08.03.1995
(21) Anmeldenummer: 92113535.6
(22) Anmeldetag: 08.08.1992
(51) Int. Cl.: B01J 23/80

(54) **Kupfer-Zinkoxid-Aluminiumoxid enthaltende Katalysatoren**
Catalysts containing copper-zincoxide-aluminiumoxide
Catalyseurs contenant cuivre, oxyde de zinc et alumine

(30) Priorität: 17.08.1991 DE 4127318
(43) Veröffentlichungstag der Anmeldung: 24.02.1993
(73) Patentinhaber: IMPERIAL CHEMICAL INDUSTRIES PLC, London SW1P 3JF (GB)
(72) Erfinder: Horn, Gerhard, Dr. Dipl.-Chem., W-4200 Oberhausen (DE); Frohning, Carl Dieter, Dr. Dipl.-Chem., W-4230 Wesel (DE)
(74) Vertreter: Gibson, Sara Hillary Margaret

(56) Entgegenhaltungen:
- EP-A- 0 125 689
- EP-A- 0 152 809
- EP-A- 0 404 408
- EP-A- 0 424 069
- EP-A- 0 482 753
- GB-A- 1 159 035
- US-A- 4 279 781
- US-A- 4 871 710
- US-A- 5 019 547

## Beschreibung

Die vorliegende Erfindung betrifft kupferhaltige Katalysatoren mit einer vergleichsweise hohen BET-Gesamtoberfläche und einer bestimmten Porenradienverteilung, ein Verfahren zu ihrer Herstellung, ausgehend von ein Kupfersalz und Salze weiterer Elemente enthaltenden wäßrigen Lösungen, aus denen mit Hilfe eines basischen Fällungsmittels ein Copräzipitat ausgefällt, das Copräzipitat anschließend gewaschen und nach Trocknung kalziniert wird, sowie ihre Verwendung.

Kupferhaltige Katalysatoren werden ebenso wie Nickelkatalysatoren in erheblichem Umfange in technischen Prozessen eingesetzt. Sie spielen beispielsweise bei Hydrierungs- und Dehydrierungsverfahren eine bedeutende Rolle. Hierbei wird der umzusetzende Einsatzstoff dem festangeordneten Katalysator entweder in gasförmigem Zustand (Gasphasefahrweise) oder in flüssigem Zustand (Flüssigphasefahrweise) zugeleitet. Der Katalysator kann auch in feinverteiltem Zustand als Aufschlämmung (Suspensionsfahrweise) genutzt werden.

Eine recht breite Anwendung haben Katalysatoren, die neben Kupfer noch Chrom enthalten, gefunden. Diese Katalysatoren sind auch unter der Bezeichnung Kupferchromit- oder Adkins-Katalysatoren bekannt.

Die Verwendung von Adkins-Katalysatoren ist allerdings nicht unproblematisch, da bei ihrer Herstellung Chrom (VI)-Verbindungen, die als krebserregend einzustufen sind und entsprechende Schutzmaßnahmen bei der Handhabung erfordern, eingesetzt werden. Zudem fallen im Verlauf der Herstellung in größeren Mengen Abwässer, die stark mit Kupfer-, Chrom (VI)- und Ammoniumverbindungen belastet sind, an Derartige Abwässer sind unerwünscht, da sowohl Kupfer- als auch Chrom (VI)-Verbindungen gegenüber Mikroorganismen hochtoxisch wirken und mittels einer aufwendigen Behandlung aus dem Abwasser entfernt werden müssen.

Neben Kupferchromit-Katalysatoren haben sich Nickelkatalysatoren in vielfacher Hinsicht bewährt. Allerdings führen Nickelkatalysatoren, insbesondere bei höheren Reaktionstemperaturen, aufgrund ihrer sehr hohen Aktivität zu unkontrollierten Neben- und Folgereaktionen, beispielsweise Spaltungen, Umalkylierungen und/oder Umlagerungen. Dies begünstigt jedoch die Bildung unerwünschter Neben- und/oder Folgeprodukte.

Es sind auch Kupferkatalysatoren, die kein chemisch gebundenes Chrom aufweisen. bekannt. Diese Katalysatoren weisen jedoch weder die Eigenschaften der Adkins-Katalysatoren, noch die von Nickelkatalysatoren auf.

So beschreibt die DE-OS 20 56 612 einen aus Mischkristallen der Reihe (CuₓZn_{y})Al₂(OH)₁₆ CO₃·4 H₂O bestehenden Katalysator, wobei x und y Zahlenwerte von 0,5 bis 5,5 annehmen können und die Summe x und y gleich 6 ist. Die Mischkristalle erhält man durch Fällung bei einem pH-Wert von 4.5 bis 5,5, indem man eine wäßrige Kupfer-, Zink- und Aluminiumnitrate enthaltende Lösung mit einem basischen Fällungsmittel, beispielsweise einer wäßrigen Na₂CO₃-Lösung, versetzt.

Der in nichtreduzierter Form CuO, ZnO und Al₂O₃ enthaltendeKatalysator wird bei der Umsetzung eines aus Kohlenmonoxid, Kohlendioxid und Wasserstoff bestehenden Gasgemisches zu Methanol eingesetzt.

Die EP 0 125 689 betrifft einen CuO, ZnO und Al₂O3 enthaltenden Katalysator für die Methanolsynthese mit einem Atomverhältnis Cu/Zn zwischen 2,8 und 3,8 (entsprechend 26,9 bis 36,5 Gew.-Teile ZnO je 100 Gew.-Teile CuO) und einem Al₂O₃-Anteil von 8 bis 12 Gew.-%. Al₂O₃ wird als kolloidales Aluminiumhydroxid in die Herstellung eingesetzt, Cu und Zn werden durch Fällung aus Metallsalzlösungen in den Katalysator eingefügt. Auf Poren mit einem Durchmesser von 2,0 bis 7,5 nm (20 bis 75 Å) - entsprechend einem Porenradius rₚ von 1,0 bis 3,75 nm (10 bis 37,5 Å) - entfallen 20 bis 40 % und auf solche mit einem Durchmesser > 7,5 nm (75 Å) - entsprechend einem Porenradius rₚ > 3,75 nm (37,5 Å) - 80 bis 60 %, bezogen jeweils auf die gesamte Anzahl der Poren. Die in den Beispielen näher beschriebenen Katalysatoren weisen in nichtreduziertem Zustand eine BET-Oberfläche von 100 bis 127 m²/g und 32 bis 42 % Poren mit einem Durchmesser von 2,0 bis 7,5 nm (rₚ = 1,0 bis 3,75 nm) und 68 bis 57 % Poren mit einem Durchmesser > 7,5 nm (rₚ > 3,75 nm) auf.

Wie die vorangegangenen Ausführungen belegen, mist ein Bedarf für einen Katalysator, der zumindest innerhalb eines bestimmten Anwendungsbereiches sowohl anstelle eines Adkins-Katalysators als auch anstelle eines Nickelkatalysators eingesetzt werden kann, vorhanden. Zudem sollen bei der Herstellung dieses Katalysators Probleme, wie Handhabung krebserregender Chrom (Vl)-Verbindungen, Anfall von Schadstoffe enthaltenden Abwässern und Entsorgung chromhaltiger Altkontakte, umgangen werden. Darüberhinaus soll der neuartige Katalysator, insbesondere bei höheren. Temperaturen, bei großer Aktivität einen hohen Umsatz und eine hohe Selektivität der Reaktion sicherstellen, das heißt, die für Nickelkatalysatoren typischen unerwünschten Neben- und Folgereaktionen sollen weitestgehend unterbunden werden.

Das gilt, unter anderem, in besonderem Maße für einen bedeutenden, sowohl Kupferchromit- respektive Adkins-Katalysatoren als auch Nickelkatalysatoren betreffenden Anwendungsbereich, nämlich die katalytische Hydrierung von Aldehyden zu den entsprechenden Alkoholen. In diesem Arbeitsgebiet soll der neuartige Katalysator nicht nur einen Nickelkatalysator, sondern auch einen Kupferchromit - respektive Adkins-Katalysator ersetzen können.

Diese Aufgabe wird gelöst durch einen Katalysator enthaltend, je 100 Gew.-Teile CuO, 40-130 Gew.-Teile ZnO, 2 bis 50 Gew.-Teile Al₂O₃ und gegebenenfalls 0.5 bis 8 Gew.-Teile Mn-, Mo-, V-, Zr- und/oder Erdalkalimetall-Oxid mit einer BET-Gesamtoberfläche von 80 bis 175 m²/g Katalysator im nichtreduzierten Zustand, wobei 75 bis 95% der BET-Gesamtoberfläche von Poren eines Radius rₚ ≦ 15 nm gebildet werden, erhältlich nach einem Verfahren, bei dem man eine Kupfer-, Zink-, Aluminium- und gegebenenfalls Mangan-, Molybdän, Vanadium-, Zirkon- und/oder Erdalkalimetall-Salze enthaltende, wäßrige Lösung, und eine wäßrige Lösung eines basischen Fällungsmittels getrennt, aber gleichzeitig zusammenführt und die Fällung bei einem konstanten pH-Wert innerhalb eines Bereiches von 7.5 bis 8.5 und oberhalb von 70, insbesondere 70 bis 95, bevorzugt 75 bis 85 °C durchführt, das anfallende Copräzipitat abtrennt, mit Wasser wäscht, trocknet, bei 320 bis 400 °C über einen Zeitraum von 4 bis 8 Stunden kalziniert und gegebenenfalls reduziert.

Ein weiteres Merkmal des Katalysators ist eine relativ große aktive Kupfermetalloberfläche. Sie beträgt im reduzierten Katalysator 30 bis 125, insbesondere 35 bis 100, bevorzugt 40 bis 85 m²/g Cu und übertrifft damit die aktive Kupfermetalloberfläche entsprechender Kupferchromit-Katalysatoren. Die Bestimmungsmethode ist M.J. Juys, P.H. van Oeffelt, W.G.J. Brouwe, A.P. Pijpers und J.J.F. Scholten, Applied Catalysis, 46 (1989), Seiten 161 bis 173 zu entnehmen.

Der Katalysator enthält je 100 Gew.-Teile CuO 40 bis 130, insbesondere 45 bis 100, bevorzugt 45 bis 80 Gew.-Teile ZnO und 2 bis 50, insbesondere 3 bis 40, bevorzugt 4 bis 30, besonders bevorzugt 4 bis 11 Gew.-Teile Al₂O₃.

Der Katalysator umfaßt erforderlichenfalls noch weitere Stoffe. Hierunter fallen Oxide des Mangans, Molybdäns, Vanadiums, Zirkons und/oder eines Erdalkalimetalls. Je 100 Gew.-Teile CuO beträgt ihr Anteile 0,5 bis 8, insbesondere 1 bis 6, bevorzugt 2 bis 4 Gew.-Teile gerechnet als MnO, MoO₃. V₂O₅, ZrO₂ und MeO, worin Me für ein Erdalkalimetall steht.

Besonders geeignet als weitere Stoffe sind Mangan- und/oder ein Erdalkalimetall-Oxid. Als Erdalkalimetall-Oxid kommen Mg-, Ca- oder Ba-Oxid, insbesondere Ca- oder Ba-Oxid, bevorzugt Ba-Oxid in Betracht.

Ein wesentliches Merkmal des erfindungsgemäßen Katalysators ist die vergleichsweise hohe BET-Gesamtoberfläche. Sie beträgt 80 bis 175, insbesondere 85 bis 160. bevorzugt 90 bis 155 m²/g Katalysator im nichtreduzierten Zustand.

Unter BET-Gesamtoberfläche wird die durch Adsorption von Stickstoff nach der Brunauer, Emmett und Teller-Methode (BET) ermittelte Oberfläche verstanden. Das Verfahren zur Bestimmung der BET-Gesamtoberfläche ist in J. Amer. Chem. Soc., 60, (1938) 309 beschrieben.

Ein wesentliches Merkmal des Katalysators ist eine bestimmte Verteilung der Porenradien. ausgedrückt durch einen hohen Anteil der BET-Gesamtoberfläche, der von Poren eines Radius rₚ ≦ 15 nm (150 Å) gebildet wird. Ihr Anteil beträgt 75 bis 95. insbesondere 80 bis 92. bevorzugt 84 bis 90 % der BET-Gesamtoberfläche.

50 bis 85, insbesondere 60 bis 80 % der BET-Gesamtoberfläche werden von Poren eines Radius rₚ ≦ 9 nm (90 Å) gebildet.

Auf den Bereich der Poren, deren Radius rₚ = 9 bis 15 nm beträgt, entfallen 5 bis 45, insbesondere 15 bis 40, bevorzugt 18 bis 30 % der BET-Gesamtoberfläche.

An dieser Stelle sei nochmals darauf hingewiesen, daß die vorangegangenen Angaben zur BET-Gesamtoberfläche sich jeweils auf den Katalysator in nichtreduzierter Form beziehen.

Die Bestimmung der Porenradien erfolgt durch Auswertung der Desorptionsisothermen mit Hilfe der Kelvin-Gleichung gemäß C. Pierce, J. Phys. Chem. 57, (1953) 149. Die Angaben hinsichtlich der Porenradien gehen ebenfalls auf den nichtreduzierten Katalysator zurück.

Der Katalysator kann, falls gewünscht, neben den bereits erwähnten Bestandteilen noch einen Träger aufweisen. Je 100 Gew.-Teile CuO enthält er 2 bis 80, insbesondere 4 bis 60, bevorzugt 5 bis 35 Gew.-Teile Träger. Als Träger können übliche in Wasser unlösliche Materialien verwendet werden. Geeignete Trägermaterialien sind SiO₂, Kieselgur, Kieselgel und Al₂O₃, insbesondere Al₂O₃.

Auf CuO, ZnO, Al₂O₃ und weitere Stoffe - hierunter sind Oxide des Mangans, des Molybdäns; des Vanadiums, des Zirkons und/oder eines Erdalkalimetalls sowie gegebenenfalls ein Träger zu verstehen - entfallen 94 bis 97 Gew.-%, bezogen auf nichtreduzierten, kalzinierten Katalysator. Die verbleibenden 3 bis 6 Gew.-% gehen auf physikalisch und/oder chemisch gebundenes Wasser, auf eventuell noch vorhandene Hydroxylgruppen, auf Oxide anderer Zusammensetzung als der Formel entsprechend, die der Berechnung zugrunde gelegt ist, auf Carbonatanteile und auf kleinere Mengen von Stoffen, die bei der Herstellung eingesetzt werden, insbesondere auf Fällungsmittel zurück.

Die Erfindung betrifft ferner einen Katalysator in reduzierter Form. Durch die Reduktion wird das CuO in metallisches Cu umgewandelt, wodurch der Katalysator aktiviert wird.

Der Katalysator enthält in reduzierter Form je 100 Gew.-Teile Cu 48 bis 163, insbesondere 56 bis 125, bevorzugt 56 bis 100 Gew.-Teile ZnO, 2.4 bis 63, insbesondere 3,7 bis 50, bevorzugt 5,0 bis 37,5, besonders bevorzugt 5 bis 13,8 Gew.Teile Al₂O₃ und gegebenenfalls 0,6 bis 10, insbesondere 1,2 bis 7,5, bevorzugt 2,4 bis 5,0 Gew.-Teile Mn-, Mo-, V-, Zr- und/oder Erdalkalimetalloxid.

Besonders geeignet sind Katalysatoren, die neben Cu, ZnO, Al₂O₃ noch Mn- oder Ba-oxid enthalten. Der Katalysator kann zusätzlich noch ein Trägermaterial aufweisen. Ein derartiger Katalysator enthält je 100 Gew.-Teile Cu 2,4 bis 100, insbesondere 4,8 bis 75, bevorzugt 6 bis 44 Gew.-Teile eines Trägers. Als Trägermaterial kommen in Wasser unlösliche Stoffe, insbesondere SiO₂ wie Kieselgur oder Kieselgel und/oder Al₂O₃, bevorzugt Al₂O₃, in Betracht.

Die übrigen Parameter, nämlich die BET-Gesamtoberfläche und die Porenradienverteilung, entsprechen in etwa den für den nichtreduzierten Katalysator ausgewiesenen Werten.

Das Verfahren zur Herstellung des erfindungsgemäßen Katalysators geht von einer wäßrigen Kupfer-, Zink-, Aluminium- und gegebenenfalls Mn-, Mo-, V-, Zr- und/oder Erdalkalimetallsalze enthaltenden, Lösung aus.
Diese, im folgenden auch als Mischsalzlösung bezeichnete Lösung enthält 10 bis 100, insbesondere 20 bis 80, bevorzugt 30 bis 60 g Cu/l, 10 bis 50, insbesondere 15 bis 40, bevorzugt 10 bis 30 g Zn/l und Al entsprechend 2 bis 80, insbesondere 5 bis 60, bevorzugt 10 bis 40 g Al₂O₃/l. Falls erforderlich weist die Mischsalzlösung noch 3 bis 80 insbesondere 5 bis 50, bevorzugt 10 bis 30 g Mn, Mo, V, Zr und/oder Erdalkalimetall, gerechnet als MnO, MoO₃, V₂O₅, ZrO₂ und MeO, worin Me für ein Erdalkalimetall steht, auf.

Die Mischsalzlösung stellt man her, indem man wasserlösliche anorganische, organische oder komplexe Salze der vorstehend genannten Elemente in Wasser löst. Gut geeignete Salze sind die Sulfate, Chloride, Acetate, Propionate, Butyrate und Nitrate. Besonders bewährt hat es sich, zur Herstellung der Mischsalzlösung Sulfate, Chloride. Acetate und Nitrate, insbesondere Nitrate zu verwenden.

Um einer partiellen, unerwünschten Hydrolyse vorzubeugen und die Fällung günstig zu beeinflussen, empfiehlt es sich, in der Mischsalzlösung einen gewissen Überschuß freier Säure aufrechtzuerhalten. Zweckmäßigerweise stellt man die Mischsalzlösung, gegebenenfalls durch Zugabe von Säure, auf einen pH-Wert unterhalb 4,5, insbesondere auf einen pH-Wert zwischen 2,0 und 4,0 ein. Dadurch wird gewährleistet, daß die Fällung unter reproduzierbaren Bedingungen abläuft, und zudem ein möglichst einheitliches Fällungsprodukt erhalten wird.

Als Säure können Mineralsäuren wie Salzsäure, Schwefelsäure und Salpetersäure eingesetzt werden. Besonders geeignet ist Salpetersäure.

Die Mischsalzlösung und die wäßrige Lösung eines basischen Fällungsmittels werden voneinander getrennt, aber gleichzeitig, gegebenenfalls in Gegenwart eines Trägers, unter Vermischung zusammengeführt.

Als Fällungsmittel dient eine wäßrige Lösung einer basischen Verbindung, insbesondere eine wäßrige Alkalicarbonat-, Alkalihydrogencarbonat-. Alkalihydroxid-, Ammoniumhydroxid- oder Ammoniumcarbonatlösung. Es können auch Mischungen derselben Anwendung finden. Besonders geeignet ist eine Na₂CO₃ und/oder NaHCO₃ enthaltende wäßrige Lösung. Das Fällungsmittel soll einen pH-Wert von 7,5 bis 13, insbesondere 8 bis 12, bevorzugt 9 bis 11 aufweisen.

Die wäßrige Lösung enthält 0,1 bis 4,0, insbesondere 0,6 bis 3,0, bevorzugt 1,6 bis 2,4 Äquivalente basische Verbindung/l Lösung. Sehr gute Ergebnisse werden mit wäßrigen Lösungen, die 0,3 bis 1,5, insbesondere 0,8 bis 1,2 Mol Alkalicarbonat/l Lösung enthalten, erzielt.

Um eine möglichst vollständige Fällung sicherzustellen und zugleich ein besonders homogenes, aus den entsprechenden basischen Kupfer-, Zink-, Aluminium-, und gegebenenfalls Mangan-, Molybdän-, Vanadium-, Zirkon und/oder Erdalkalimetallverbindungen bestehendes Copräzipitat zu erhalten, setzt man die basische Verbindung im Überschuß ein. Im allgemeinen beträgt der stöchiometrische Überschuß 5 bis 60, insbesondere 10 bis 50 % basische Verbindung. Besonders bewährt hat sich die Verwendung von 15 bis 30 % überschüssige basische Verbindung. Der Überschuß bezieht sich jeweils auf die Menge basische Verbindung, die zur vollständigen Fällung der in der Mischsalzlösung enthaltenen Metalle erforderlich ist.

Der stöchiometrische Überschuß ist derart zu bemessen, daß einerseits zwar die Fällung eines möglichst homogenen Copräzipitats gewährleistet ist, andererseits aber auch eine Fällung der in der Mischsalzlösung enthaltenen Metallionen im jeweils gewünschten Umfange eintritt, wobei einer weitestgehend vollständigen Fällung der Vorzug zu geben ist.

Die Fällung wird herbeigeführt, indem man die Mischsalzlösung und das Fällungsmittel voneinander getrennt, aber gleichzeitig, kontinuierlich oder diskontinuierlich unter intensiver Vermischung zusammenführt.

Die Fällung des aus basischen Verbindungen bestehenden Copräzipitats wird durch vergleichsweise langsames Zusammenführen der Mischsalzlösung und des Fällungsmittels herbeigeführt. Die Fällungszeit sollte wenigstens 10, insbesondere wenigstens 15, bevorzugt wenigstens 20 Minuten betragen.

Die Mischsalzlösung und die Lösung des basischen Fällungsmittels werden auf eine vorgegebene Temperatur erhitzt. Diese Temperatur sollte in etwa der Temperatur, bei der die Fällung durchgeführt wird, entsprechen.

In den meisten Fällen hat es sich bewährt, eine Fällungszeit von 10 bis 100, insbesondere 15 bis 95, bevorzugt 20 bis 90 Minuten einzuhalten. Die Fällungszeit läßt sich durch Wahl einer entsprechenden Zugabegeschwindigkeit von Mischsalzlösung und Fällungsmittel, insbesondere bei diskontinuierlich durch-geführter Fällung, einstellen, während bei kontinuierlicher Arbeitsweise neben den Zugabegeschwindigkeiten von Mischsalzlösung und Fällungsmittel auch noch der auf das Reaktionsvolumen bezogene Durchsatz zu berücksichtigen ist.

Man arbeitet während der Fällung bei einem konstanten pH-Wert innerhalb eines pH-Bereiches von 7,5 bis 8.5, Schwankungen des pH-Wertes sollten möglichst gering gehalten werden, um die Herstellung eines besonders homogenen Copräzipitats zu gewährleisten. Die Schwankungen des pH-Wertes sollten auf ein Intervall von -0,5 bis +0,5, vorzugsweise -0,2 bis +0.2 begrenzt werden.

Die Fällung wird bei Temperaturen oberhalb 70°C, insbesondere in einem Bereich von 75 bis 95, bevorzugt 75 bis 85°C durchgeführt. Hierzu genügt es, die an der Fällung beteiligten Stoffe, nämlich die Mischsalzlösung und das Fällungsmittel auf eine entsprechend hohe Temperatur einzustellen. Während der Fällung sollte die Temperatur möglichst konstant gehalten werden. Die Abweichungen von der vorgegebenen Temperatur sollten ± 2, insbesondere ± 1 ° C nicht übersteigen. Größere Schwankungen der Fällungstemperatur können die Beschaffenheit, beispielsweise Partikelgröße, Homogenität und Struktur des Copräzipitats in nachteiliger Weise beeinflussen.

Im Anschluß an die Fällung wird das Copräzipitat, gegebenenfalls nach kurzem Nachrühren, von der Mutterlauge, beispielsweise durch Dekantieren und/oder Filtrieren, abgetrennt und sorgfältig gewaschen. Hierbei beeinflussen die verwendete Menge Waschwasser, die Strömungsgeschwindigkeit und Temperatur des Waschwasser sowie die Dauer des Waschvorganges das Entfernen unerwünschter Bestandteile aus dem Copräzipitat, insbesondere das Herauslösen von Nitrat- und Alkaliionen.

Steigende Mengen Waschwasser, hohe Temperaturen des Waschwassers und lange Dauer des Waschvorganges fördern ein besonders weitgehendes Herauslösen, während niedrigere Mengen Waschwasser, niedrige Temperaturen des Waschwassers und kurze Dauer des Waschvorganges zu einem verringerten Herauslösen führen.

Im allgemeinen genügt es, während des Waschens eine Temperatur von 55 bis 85, insbesondere 60 bis 75 ° C einzuhalten. Höhere Waschtemperaturen können sich auf die Qualität des Präzipitats nachteilig auswirken. Zu niedrige Temperaturen können dazu führen, daß die unerwünschten Bestandteile nicht im erforderlichen Ausmaße aus dem Copräzipitat entfernt werden. Auch dies kann sich auf die Qualität des Wertproduktes ungünstig auswirken.

Üblicherweise verwendet man 5 bis 50 insbesondere 10 bis 30, bevorzugt 15 bis 25 Liter Waschwasser je kg Copräzipitat.

Die Dauer des Waschvorganges muß ausreichend bemessen werden. Sie sollte mindestens 60, insbesondere mindestens 70, bevorzugt mindestens 80 Minuten betragen. Üblicherweise genügt eine Waschdauer von 80 bis 140, insbesondere 85 bis 130, bevorzugt 90 bis 120 Minuten.

Man kann den Waschprozeß in einem einzigen Schritt durchführen oder ihn in mehreren, aufeinanderfolgenden Stufen ablaufen lassen, indem man die gesamt erforderliche Menge Waschwasser entsprechend verteilt. Welchem Vorgehen man den Vorzug gibt, hängt vom Einzelfall ab. Im allgemeinen führt sowohl ein einstufiger als auch ein mehrstufiger Waschvorgang zum gewünschten Erfolg. Ein mehrstufiger Waschprozeß kann in vielen Fällen vorteilhaft sein, da meist weniger Waschwasser als bei einem einstufigen Prozess benötigt wird.

Eine Kontrolle des Waschvorganges wird durch Bestimmung der im Waschwasser vorhandenen, herausgelösten Verbindungen, wie NaNO₃, erleichtert. Auf eine derartige Überwachung kann verzichtet werden, falls man den Waschvorgang unter standardisierten und kontrollierten Bedingungen, beispielsweise bei vorgegebener Menge Waschwasser, Temperatur und Dauer, ausübt.

Falls gewünscht, kann man das gewaschene Material in stückige Form bringen. Zur Formgebung kann man sich bewährter Verfahren, beispielsweise der Strangextrusion, bedienen.

Die Trocknung wird bei erhöhten Temperaturen, vorzugsweise bei steigenden Temperaturen stufenweise durchgeführt. Es erweist sich als ausreichend, die Trocknung bei Temperaturen von 50 bis 120, insbesondere 55 bis 100, bevorzugt 60 bis 90°C unter Anwendung üblicher Verfahren, wie Anordnung des Trockengutes im Festbett oder im bewegten Bett, beispielsweise in Wirbelschicht, durchzuführen.

Man trocknet, bis ein Restfeuchtegehalt von etwa 2 bis 15, insbesondere 3 bis 10 Gew.-% Wasser, bezogen auf das getrocknete Copräzipitat, erreicht wird.

Die anschließende Kalzinierung erfolgt bei 320 bis 400°C über einen Zeitraum von 4 bis 8 Stunden. Der kalzinierte Katalysator kann entweder in Pulverform direkt für Suspensionshydrierungen oder nach Formgebung, wie Tablettierung oder Pelletierung, als festangeordneter Katalysator eingesetzt werden.

Aus dem kalzinierten Katalysator wird durch Reduktion (Aktivierung) der eigentliche hydrieraktive Katalysator hergestellt. Zu diesem Zweck behandelt man den kalzinierten, nichtreduzierten Katalysator bei 130 bis 190°C. gegebenenfalls unter erhöhtem Druck, mit Wasserstoff. Besonders bewährt hat es sich, die Reduktion mittels eines Wasserstoff-Stickstoffgemisches durchzuführen. Es empfiehlt sich, ein Wasserstoff-Stickstoff-Gemisch mit 1 bis 5 Vol.-% Wasserstoff zu verwenden. Durch die Behandlung mit Wasserstoff bildet sich aus CuO metallisches Cu.

Der erfindungsgemäße Katalysator läßt sich vorteilhaft für Hydrierungen von Aldehyden, wie aromatischen und aliphatischen geradkettigen und/oder verzweigten Aldehyden, in flüssiger, aber auch in gasförmiger Phase anwenden. Ein Vorteil besteht darin, hohe Umsätze und gute Selektivitäten selbst bei vergleichsweise hohen Temperaturen zu erzielen, ohne eine Bildung unerwünschter Nebenprodukte in nennenswerten Maße in Kauf nehmen zu müssen. Auf diese Weise lassen sich auch reaktionsträge Aldehyde und Aldehydderivate, beispielsweise ungesättigte aliphatische Aldehyde, problemlos zu den entsprechenden Alkoholen hydrieren.

Die nachfolgenden Beispiele belegen die vorliegende Erfindung, ohne sie zu begrenzen.

### Experimenteller Teil

### Beispiel 1

### Herstellung eines CuO, ZnO und Al₂O₃ enthaltenden Katalysators.

Man löst 4 356 g Cu(NO₃)₂ . 3 H₂O, 2 615 g Zn(NO₃)₂ . 6 H₂O und 895,8 g Al(NO₃)₃ . 9 H₂O in 18 Liter deionisiertem Wasser (Mischsalzlösung). Die Mischsalzlösung wird auf 80 °C erhitzt.

Die Lösung des basischen Fällungsmittels erhält man, indem man 3 750 g Na₂CO₃ in 30 Liter deionisiertem Wasser löst. Diese Lösung wird ebenfalls auf 80 ° C erhitzt.

In einem Fällungsbehälter, der mit einem Rührwerk bestückt ist. werden 8 Liter deionisiertes Wasser vorgelegt und unter Rühren auf 80 ° C erhitzt. Man läßt die Mischsalzlösung und die Lösung des basischen Fällungsmittels getrennt, aber gleichzeitig innerhalb von 20 Minuten in den Fällungsbehälter unter kräftigem Rühren einlaufen. Man stimmt die Zulaufgeschwindigkeiten der beiden Lösungen so aufeinander ab, daß im Fällungsbehälter ein pH-Wert von 7,5 bis 7,8 eingehalten wird. Die Temperatur im Fällungsbehälter wird bei 80 ° C konstant gehalten.

Nach Abschluß der Fällung wird die im Fällungsbehälter vorliegende Suspension noch 2 Minuten gerührt. Anschließend wird der Niederschlag (Copräzipitat) durch Filtration von der Mutterlauge abgetrennt und nachfolgend innerhalb von 2 Stunden mit 120 Liter 60 bis 65°C heißem Wasser gewaschen. Der gewaschene Filterkuchen wird entweder in extrudierter Form oder durch Sprühtrocknung auf eine Endfeuchte von ≦ 5 Gew.-% H₂O, bezogen auf Katalysatormasse, getrocknet.

Das getrocknete Fällungsprodukt wird 4 Stunden im N₂-Strom bei 380 ° C kalziniert.

Der kalzinierte Katalysator weist 47,5 Gew.-% Cu auf. Er enthält je 100 Gew.-Teile CuO 49,5 Gew.-Teile ZnO, 8,4 Gew.-Teile Al₂O₃, 3,7 Gew.-Teile CO₂ und 0,12 Gew.-Teile Na₂O.

Die BET-Gesamtoberfläche beträgt 128 m²/g Katalysator im nichtreduzierten Zustand. 85 % der BET-Gesamtoberfläche werden von Poren eines Radius rₚ ≦ 15 nm gebildet. Die Kupfermetalloberfläche des reduzierten Katalysators liegt bei 68 m²/g Cu.

### Beispiel 2

### Herstellung eines CuO, ZnO, Al₂O₃ und BaO enthaltenden Katalysators.

Man löst 4 356 g Cu(NO₃)₂ . 3 H₂O, 2 615 g Zn(NO₃)₂ . 6 H₂O, 895,8 g Al(NO₃)₃ . 9 H₂O und 73,2 g Ba(NO₃)₂ in 18 Liter deionisiertem Wasser (Mischsalzlösung). Die Mischsalzlösung wird auf 80° C erhitzt.

Die Lösung des basischen Fällungsmittels erhält man, indem man 3 750 g Na₂GO₃ in 30 Liter deionisiertem Wasser löst. Diese Lösung wird ebenfalls auf 80 °C erhitzt.

Anschließend arbeitet man genau wie in Beispiel 1 angegeben.

Der kalzinierte Katalysator weist 46,5 Gew.-% Cu auf. Er enthält je 100 Gew.-Teile CuO 49,8 Gew.-Teile ZnO, 8,2 Gew.-Teile Al₂O₃, 3,6 Gew.-Teile CO₂, 0,12 Gew.-Teile Na₂O und 2,0 Gew.-Teile BaO. Die BET-Gesamtoberfläche beträgt 127 m²/g Katalysator im nichtreduzierten Zustand. 88 % der BET-Gesamtoberfläche werden von Poren eines Radius rₚ ≦ 15 nm gebildet. Die Kupfermetalloberfläche des reduzierten Katalysators liegt bei 65 m²/g Cu.

### Beispiel 3

### Herstellung eines CuO, ZnO, Al₂O₃ und MnO enthaltenden Katalysators.

Man löst 1 936 g Cu(NO₃)₂ . 3 H₂O, 1 152,4 g Zn(NO₃)₂ . 6 H₂O, 234,2 g Al(NO₃)₃ . 9 H₂O und 78.99 Mn(NO₃)₂ . 4 H₂O in 10,2 Liter deionisiertem Wasser (Mischsalzlösung). Die Mischsalzlösung wird auf 80°C erhitzt.

Die Lösung des basischen Fällungsmittels erhält man, indem man 1 620 g Na₂CO₃ in 15,4 Liter deionisiertem Wasser löst. Diese Lösung wird ebenfalls auf 80 ° C erhitzt.

In einem Fällungsbehalter, der mit einem Rührwerk bestückt ist, werden 6 Liter deionisiertes Wasser vorgelegt und unter Rühren auf 80 ° C erhitzt. Man läßt die Mischsalzlösung und die Lösung des basischen Fällungsmittels getrennt, aber gleichzeitig innerhalb von 17 Minuten in den Fällungsbehälter unter kräftigem Rühren einlaufen.

Anschließend arbeitet man genau wie in Beispiel 1 angegeben.

Der kalzinierte Katalysator weist 47,3 Gew.-% Cu auf. Er enthält je 100 Gew.-Teile CuO 53,3 Gew.-Teile ZnO, 5,3 Gew.-Teile Al₂O₃, 2,9 Gew.-Teile MnO, 3,2 Gew.-Teile CO₂ und 0,15 Gew.-Teile Na₂O.

Die BET-Gesamtoberfläche beträgt 95 m²/g Katalysator im nichtreduzierten Zustand. 85 % der BET-Gesamtoberfläche werden von Poren eines Radius rₚ ≦ 15 nm gebildet. Die Kupfermetalloberfläche des reduzierten Katalysators liegt bei 54 m²/g Cu.

### Beispiel 4

### Herstellung eines CuO, ZnO. Al₂O₃, MnO und Träger enthaltenden Katalysators.

Man löst 1 459 g Cu(NO₃)₂ . 3 H₂O, 700,7 g Zn(NO₃)₂ . 6 H₂O, 176,5/g Al(NO₃)₃ . 9 H₂O und 59,4 g Mn(NO₃)₂ . 4 H₂O in 7,7 Liter deionisiertem Wasser (Mischsalzlösung). Die Mischsalzlösung wird auf 85°C erhitzt.

Die Lösung des basischen Fällungsmittels erhält man. indem man 1 505 g Na₂CO₃ in 14,2 Liter deionisiertem Wasser löst. Die Lösung wird ebenfalls auf 85 ° C erhitzt.

In einem Fällungsbehälter, der mit einem Rührwerk bestückt ist, werden als Träger 48 g feinteiliges Al₂O₃ (Handelsprodukt "Type H" der Firma Martinswerk/Bergheim) und 4 Liter deionisiertes Wasser vorgelegt und unter Rühren auf 85 ° C erhitzt. Man läßt die Mischsalzlösung und die Lösung des basischen Fällungsmittels getrennt, aber gleichzeitig innerhalb von 24 Minuten in den Fällungsbehälter unter kräftigem Rühren einlaufen. Man stimmt die Zulaufgeschwindigkeiten der beiden Lösungen so aufeinander ab, daß im Fällungsbehälter ein pH-Wert von 7,7 bis 8,0 eingehalten wird. Die Temperatur im Fällungsbehälter wird bei 85°C konstant gehalten. Nach Abschluß der Fällung wird wie in Beispiel 1 angegeben gearbeitet, das getrocknete trägerhaltige Fällungsprodukt allerdings 5 Stunden im N₂-Strom bei 380 ° C kalziniert.

Der kalzinierte Katalysator weist 45,6 Gew.-% Cu auf. Er enthält je 100 Gew.-Teile CuO 43.2 Gew.-Teile ZnO, 13,3 Gew.-Teile Al₂O₃, 2,8 Gew.-Teile MnO, 3,7 Gew.-Teile CO₂ und 0,1 Gew.-Teile Na₂O. Die BET-Gesamtoberfläche beträgt 117 m²/g nichtreduzierten Katalysator. 83 % der BET-Gesamtoberfläche werden von Poren eines Radius rₚ ≦ 15 nm gebildet. Die Kupfermetalloberfläche des reduzierten Katalysators liegt bei 76 m²/g Cu.

### Beispiel 5

### Hydrierung von n-Butanal

In einem Doppelmantel-Rohrreaktor mit einem Innendurchmesser von 38 mm werden 3 Liter des nach Beispiel 1 hergestellten Katalysators in tablettierter Form (6 x 5 mm Tabletten) eingefüllt.

Man erhitzt die Katalysator enthaltende Schicht auf 160°C und leitet zur Aktivierung einen 3 Vol.-% H₂ enthaltenden N₂-Strom bei Normaldruck mit einer Gasvolumengeschwindigkeit von 1 000 1/Liter Katalysator x Stunde (V_{Gasstrom}/V_{Katalysator} x h) durch die Katalysatorschicht. Nach 24 Stunden beendet man die Zufuhr des 3 Vol.-% H₂ enthaltenden N₂-Stromes und senkt die Temperatur in der Katalysatorschicht auf 143°C ab.

In einem bei 130°C betriebenen Verdampfer wird n-Butanal unter Zuleiten eines H₂-Gasstromes (98 %iger Wasserstoff), der auf einen Druck von 0,35 MPa und eine Strömungsgeschwindigkeit von 10,2 Nm³/h eingestellt wird, verdampft. Das resultierende Gasgemisch wird in einem Erhitzer auf 143°C aufgeheizt und dem in dem Doppelmantel-Rohrreaktor festangeordneten Katalysator zugeleitet.

Es werden über einen Zeitraum von je 8 Stunden zunächst 600, 1 200 und 1 800 ml flüssiges n-Butanal/h eingesetzt (entsprechend einer V/Vh von 0,2, 0,4 und 0,6 wobei V/Vh für Volumen flüssiges n-Butanal/Volumen Katalysator x Stunde steht). Anschließend werden je Stunde 3 000 ml flüssiges n-Butanal (V/Vh = 1) der Umsetzung (in verdampfter Form) zugeleitet.

Das anfallende Reaktionsprodukt weist die nachfolgende, durch gaschromatographische Analyse ermittelte Zusammensetzung auf.
n-Butanal 0,1 bis 0,3 Gew.-%
n-Butanol 99,5 bis 99,7 Gew.-%
i-Butanol 0,04 Gew.-%
Rest Nebenprodukte
Die CO-Zahl beträgt 1.0 [mg KOH/g].

### Beispiel 6

### Hydrierung von 2-Ethylhexenal

In einem Doppelmantel-Rohrreaktor mit einem Innendurchmesser von 38 mm werden 3 Liter des nach Beispiel 4 hergestellten Katalysators in tablettierter Form (6 x 5 mm Tabletten) eingefüllt.

Der Katalysator wird, wie in Beispiel 5 angegeben, aktiviert und anschließend die Temperatur in der Katalysatorschicht auf 143°C abgesenkt.

In einem bei 100°C betriebenen Verdampfer werden 1 500 ml 2-Ethylhexenal/Stunde (entsprechend V/Vh = 0,5, wobei V/Vh für Volumen flüssiges 2-Ethylhexenal/Volumen Katalysator x Stunde steht) unter Zuleiten eines H₂-Stromes (98 %iger Wasserstoff), der auf einen Druck von 0,15 MPa und eine Strömungsgeschwindigkeit von 4,4 Nm³/h eingestellt wird, verdampft. Das resultierende Gasgemisch wird in einem Erhitzer auf 140°C aufgeheizt und dem in dem Doppelmantel-Rohreaktor festangeordneten Katalysator zugeleitet.

Die Umsetzung wird 2 160 Stunden ohne Unterbrechung durchgeführt, ohne daß eine merkliche Beeinträchtigung des Reaktionsablaufes festzustellen war.

Die Zusammensetzung des Einsatzmaterials und des Reaktionsproduktes (ermittelt durch gaschromatographische Analyse) sind in der nachfolgenden Tabelle zusammengestellt.

**Tabelle**

| | Einsatzmaterial (in Gew.-%) | Reaktionsprodukt (in Gew-%) |
|---|---|---|
| C₃-C₇-Kohlenwasserstoff | 0,1 | 0,3 - 0,4 |
| n-Butanal | 1,6 | - |
| n/i-Butanol | 0,7 | 2,2 |
| 2-Ethylhexanal | 0,6 | 0,4 |
| 2-Ethylhexenal | 91,0 | <0,01 |
| 2-Ethylhexanol | 4,0 | 96,5 |
| Höhersieder | ca. 1,5 | ca. 0,5 |

## Patentansprüche

1. Katalysator enthaltend, je 100 Gew.-Teile CuO, 40-130 Gew.-Teile ZnO, 2 bis 50 Gew.-Teile Al₂O₃ und gegebenenfalls 0.5 bis 8 Gew.-Teile Mn-, Mo-, V-, Zr- und/oder Erdalkalimetall-Oxid mit einer BET-Gesamtoberfläche von 80 bis 175 m²/g Katalysator im nichtreduzierten Zustand, wobei 75 bis 95% der BET-Gesamtoberfläche von Poren eines Radius rₚ ≤ 15 nm gebildet werden, erhältlich nach einem Verfahren, bei dem man eine Kupfer-, Zink-, Aluminium- und gegebenenfalls Mangan-, Molybdän, Vanadium-, Zirkon- und/oder Erdalkalimetall-Salze enthaltende, wäßrige Lösung, und eine wäßrige Lösung eines basischen Fällungsmittels getrennt, aber gleichzeitig zusammenführt und die Fällung bei einem konstanten pH-Wert innerhalb eines Bereiches von 7.5 bis 8.5 und oberhalb von 70, insbesondere 70 bis 95, bevorzugt 75 bis 85 °C durchführt, das anfallende Copräzipitat abtrennt, mit Wasser wäscht, trocknet, bei 320 bis 400 °C über einen Zeitraum von 4 bis 8 Stunden kalziniert und gegebenenfalls reduziert.

2. Katalysator nach Anspruch 1, **dadurch gekennzeichnet, daß** die aktive Kupfermetalloberfläche des reduzierten Katalysators 30 bis 125, insbesondere 35 bis 100, bevorzugt 40 bis 85 m²/g Cu, beträgt.

3. Katalysator nach Anspruch 1 oder 2, **dadurch gekennzeichnet, daß** er, je 100 Gew.-Teile CuO, 45 bis 100, insbesondere 45 bis 80 Gew.-Teile ZnO und 3 bis 40, insbesondere 4 bis 30, bevorzugt 4 bis 11 Gew.-Teile Al₂O₃ enthält.

4. Katalysator nach einem oder mehreren der Ansprüche 1 bis 3, **dadurch gekennzeichnet, daß** er, je 100 Gew.-Teile CuO, 0,5 bis 8, insbesondere 1 bis 6, bevorzugt 2 bis 4 Gew.-Teile Mn-, Mo-, V-, Zr- und/oder Erdalkalimetall-Oxid, insbesondere Mn und/oder Erdalkalimetall-Oxid enthält.

5. Katalysator nach einem oder mehreren der Ansprüche 1 bis 4, **dadurch gekennzeichnet, daß** er als Erdalkalimetall-Oxid Mg-, Ca- oder Ba-Oxid, insbesondere Ca- oder Ba-Oxid, bevorzugt Ba-Oxid enthält.

6. Katalysator nach einem oder mehreren der Ansprüche 1 bis 5, **dadurch gekennzeichnet, daß** die BET-Gesamtoberfläche 85 bis 160. insbesondere 90 bis 155 m²/g Katalysator im nichtreduzierten Zustand beträgt.

7. Katalysator nach einem oder mehreren der Ansprüche 1 bis 6, **dadurch gekennzeichnet, daß** 80 bis 92. vorzugsweise 84 bis 90 % der BET-Gesamtoberfläche von Poren eines Radius rₚ ≦ 15 nm gebildet werden.

8. Katalysator nach einem oder mehreren der Ansprüche 1 bis 7, **dadurch gekennzeichnet, daß** 50 bis 85, insbesondere 60 bis 80 % der BET-Gesamtoberfläche von Poren eines Radius rₚ ≦ 9 nm gebildet werden.

9. Katalysator nach einem oder mehreren der Ansprüche 1 bis 8, **dadurch gekennzeichnet, daß** 5 bis 45, insbesondere 15 bis 40, bevorzugt 18 bis 30 % der BET-Gesamtoberfläche von Poren eines Radius rₚ = 9 bis 15 nm gebildet werden.

10. Katalysator nach einem oder mehreren der Ansprüche 1 bis 9, **dadurch gekennzeichnet, daß** er, je 100 Gew.-Teile CuO, zusätzlich 2 bis 80, insbesondere 4 bis 60, bevorzugt 5 bis 35 Gew.-Teile eines in Wasser unlöslichen Trägers, insbesondere SiO₂ wie Kieselgur oder Kieselgel und/oder Al₂O₃, bevorzugt Al₂O₃ enthält.

11. Katalysator nach einem oder mehreren der Ansprüche 1 bis 10, **dadurch gekennzeichnet, daß** er in reduzierter Form je 100 Gew.-Teile Cu, 48 bis 163 Gew.-Teile ZnO, 2,4 bis 63 Gew.-Teile Al₂O₃ und gegebenenfalls 0,6 bis 10 Gew.-Teile Mn-, Mo-, V-, Zr- und/oder Erdalkalimetalloxid enthält.

12. Katalyator nach Anspruch 11, **dadurch gekennzeichnet, daß** er je 100 Gew.-Teile Cu 56 bis 125, insbesondere 56 bis 100 Gew.-Teile ZnO, 3,7 bis 50, insbesondere 5,0 bis 37,5 bevorzugt 5,0 bis 13.8 Gew.-Teile Al₂O₃ und gegebenenfalls 1,2 bis 7,5, insbesondere 2,4 bis 5.0 Gew.-Teile Mn-, Mo-, V-, Zr- und/oder Erdalkalimetalloxid enthält.

13. Katalysator nach Anspruch 11 oder 12, **dadurch gekennzeichnet, daß** er Mn- oder Ba-oxid enthält.

14. Katalysator nach einem oder mehreren der Ansprüche 11 bis 13, **dadurch gekennzeichnet, daß** er je 100 Gew.-Teile Cu 2,4 bis 100, insbesondere 4,8 bis 75, bevorzugt 6 bis 44 Gew.-Teile eines in Wasser unlöslichen Trägers, insbesondere SiO₂, Kieselgur, Kieselgel, Al₂O₃, bevorzugt Al₂O₃ enthält.

15. Verfahren zur Herstellung eines Katalysators nach einem oder mehreren der Ansprüche 1 bis 14, **dadurch gekennzeichnet, daß** man eine Kupfer-, Zink-, Aluminium- und gegebenenfalls Mangan-, Molybdän, Vanadium-, Zirkon- und/oder Erdalkalimetall-Salze enthaltende, wäßrige Lösung und eine wäßrige Lösung eines basischen Fällungsmittels getrennt, aber gleichzeitig zusammenführt und die Fällung, gegebenenfalls in Gegenwart eines Trägers, bei einem konstanten pH-Wert innerhalb eines Bereiches von 7.5 bis 8.5 und oberhalb von 70, insbesondere 70 bis 95 bevorzugt 75 bis 85 °C durchführt, das anfallende Copräzitpitat abtrennt, mit Wasser wäscht, trocknet, bei 320 bis 400 °C über einen Zeitraum von 4 bis 8 Stunden kalziniert und gegebenenfalls reduziert.

16. Verfahren zur Herstellung des Katalysators nach Anspruch 15, **dadurch gekennzeichnet, daß** man den kalzinierten Katalysator bei 130 bis 190°C, gegebenenfalls unter erhöhtem Druck, mit einem Wasserstoff-Stickstoff-Gemisch, das 1,0 bis 5 Vol.-% Wasserstoff enthält, reduziert.

17. Verwendung des Katalysators zur Hydrierung von Aldehyden zu Alkoholen.

## Claims

1. A catalyst containing, per 100 parts by weight of CuO, 40 to 130 parts by weight of ZnO, 2 to 50 parts by weight of Al₂O₃ and optionally, 0.5 to 8 parts by weight of an oxide of Mn, Mo, V, Zr and/or of an alkaline earth metal, having a BET total surface area of 80 to 175 m²/g of catalyst in the unreduced state, 75 to 95% of the BET total surface area being formed by pores having a radius rₚ ≤ 15 nm obtainable by a process in which an aqueous solution containing salts of copper, zinc, aluminium and optionally of manganese, molybdenum, vanadium, zirconium and/or of an alkaline earth metal, and an aqueous solution of a basic precipitant are separately but simultaneously brought together and the precipitation is carried out at a constant pH within a range of 7.5 to 8.5 and above 70 °C, in particular 70 to 95 °C, preferably 75 to 85 °C, the coprecipitate produced is separated off, washed with water, dried, calcined at from 320 to 400 °C over a period of from 4 to 8 hours and reduced if required.

2. A catalyst as claimed in claim 1, **characterised in that** the active copper metal surface area of the reduced catalyst is 30 to 125, in particular 35 to 100, preferably 40 to 85 m²/g of Cu.

3. A catalyst as claimed in claim 1 or 2, which contains, per 100 parts by weight of CuO, 45 to 100, in particular 45 to 80 parts by weight of ZnO and 3 to 30, in particular 4 to 30, preferably 4 to 11 parts by weight of Al₂O₃.

4. A catalyst as claimed in one or more of claims 1 to 3, which contains, per 100 parts by weight of CuO, 0.5 to 8, in particular 1 to 6, preferably 2 to 4 parts by weight of an oxide of Mn, Mo, V, Zr and/or of an alkaline earth metal, in particular an oxide of Mn and/or of an alkaline earth metal.

5. A catalyst as claimed in one or more of claims 1 to 4, wherein as alkaline earth metal oxide it contains magnesium oxide, calcium oxide or barium oxide, in particular calcium oxide or barium oxide, preferably barium oxide.

6. A catalyst as claimed in one or more of claims 1 to 5, wherein the BET total surface area is 85 to 160, in particular 90 to 155 m²/g of catalyst in the unreduced state.

7. A catalyst as claimed in one or more of claims 1 to 6, wherein 80 to 92%, preferably 84 to 90% of the BET total surface area is formed by pores having a radius rₚ ≤15 nm.

8. A catalyst as claimed in one or more of claims 1 to 7, wherein 50 to 85%, in particular 60 to 80% of the BET total surface area is formed by pores having a radius rₚ ≤ 9 nm.

9. A catalyst as claimed in one or more of claims 1 to 8, wherein 5 to 45%, in particular 15 to 40%, preferably 18 to 30% of the BET total surface area is formed by pores having a radius rₚ = 9 to 15nm.

10. A catalyst as claimed in one or more of claims 1 to 9, which contains, per 100 parts by weight of CuO, in addition 2 to 80, in particular 4 to 60, preferably 5 to 35 parts by weight of a support which is insoluble in water, in particular SiO₂ such as kieselguhr or silica gel and/or Al₂O₃, preferably Al₂O₃.

11. A catalyst as claimed in one or more of claims 1 to 10, which, in reduced form, contains, per 100 parts by weight of Cu, 48 to 163 parts by weight of ZnO, 2.4 to 63 parts by weight of Al₂O₃ and possibly 0.6 to 10 parts by weight of an oxide of Mn, Mo, V, Zr and/or of an alkaline earth metal.

12. A catalyst as claimed in claim 11, which contains, per 100 parts by weight of Cu, 56 to 125, in particular 56 to 100 parts by weight of ZnO, 3.7 to 50, in particular 5.0 to 37.5, preferably 5.0 to 13.8 parts by weight of Al₂O₃ and possibly 1.2 to 7.5, in particular 2.4 to 5.0 parts by weight of an oxide of Mn, Mo, V, Zr and/or of an alkaline earth metal.

13. A catalyst as claimed in claim 11 or 12, which contains manganese oxide or barium oxide.

14. A catalyst as claimed in one or more of claims 11 to 13, wherein it contains, per 100 parts by weight of Cu, 2.4 to 100, in particular 4.8 to 75, preferably 6 to 44 parts by weight of a support which is insoluble in water, in particular SiO₂ kieselguhr, silica gel, Al₂O₃ preferably Al₂O₃.

15. A process for the preparation of a catalyst as claimed in one or more of claims 1 to 14, wherein an aqueous solution containing salts of copper, zinc, aluminium and optionally of manganese, molybdenum, vanadium, zirconium and/or of an alkaline earth metal, and an aqueous solution of a basic precipitant are separately but simultaneously brought together and the precipitation, optionally in the presence of a support, is carried out at a constant pH within a range of 7.5 to 8.5 and above 70°C, in particular 70 to 95 °C, preferably 75 to 85 °C, the coprecipitate produced is separated off, washed with water, dried, calcined at from 320 to 400 °C over a period of from 4 to 8 hours and reduced if required.

16. A process for the preparation of a catalyst as claimed in claim 15, wherein the calcined catalyst is reduced at 130 to 190°C, possibly under elevated pressure, with a hydrogen/nitrogen mixture, which contains 1.0 to 5% by volume of hydrogen.

17. The use of the catalyst for the hydrogenation of aldehydes to give alcohols

## Revendications

1. Catalyseur contenant, pour 100 parties en poids de CuO, 40 à 130 parties en poids de ZnO, 2 à 50 parties en poids de Al₂O₃ et éventuellement 0,5 à 8 parties en poids d'un oxyde de Mn, de Mo, de V, de Zr et/ou d'un métal alcalino-terreux ayant une surface spécifique totale BET de 80 à 175 m²/g à l'état non réduit, 75 à 95% de la surface spécifique totale BET étant formée par des pores d'un rayon rₚ inférieur ou égal à 15 nm, susceptible d'être obtenu selon un procédé où on combine, en les introduisant séparément mais simultanément, une solution aqueuse contenant des sels de cuivre, de zinc, d'aluminium et éventuellement de manganèse, de molybdène, de vanadium, de zirconium et/ou d'un métal alcalino-terreux et une solution aqueuse d'un agent basique de précipitation et l'on effectue la précipitation, à une valeur constante du pH comprise dans un intervalle allant de 7,0 à 8,5, et au-dessus de 70, en particulier de 70 à 95, de préférence de 75 à 85°C, on sépare le co-précipité obtenu, on le lave à l'eau, on le sèche, on le calcine entre 320 et 400°C en un espace de temps de 4 à 8 heures et éventuellement on le réduit.

2. Catalyseur selon la revendication 1, **caractérisé en ce que** la surface spécifique du cuivre métallique actif du catalyseur réduit représente 30 à 125, notamment 35 à 100, de préférence 40 à 85 m²/g de Cu.

3. Catalyseur selon la revendication 1 ou 2, **caractérisé en ce qu'**il contient, pour 100 parties en poids de CuO, 45 à 100, notamment 45 à 80 parties en poids de ZnO et 3 à 40, notamment 4 à 30, de préférence 4 à 11 parties de Al₂O₃.

4. Catalyseur selon l'une ou plusieurs des revendications 1 à 3, **caractérisé en ce qu'**il contient, pour 100 parties en poids de CuO, 0,5 à 8, notamment 1 à 6, de préférence 2 à 4 parties en poids d'un oxyde de Mn, Mo, V, Zr et/ou un métal alcalino-terreux, notamment de l'oxyde de Mn et/ou d'un métal alcalino-terreux.

5. Catalyseur selon l'une ou plusieurs des revendications 1 à 4, **caractérisé en ce qu'**il contient, comme oxyde de métal alcalino-terreux, de l'oxyde de Mg, Ca ou Ba, notamment de l'oxyde de Ca ou Ba et de préférence de l'oxyde de Ba.

6. Catalyseur selon l'une ou plusieurs des revendications 1 à 5, **caractérisé en ce que** la surface spécifique totale BET vaut 85 à 160, notamment 90 à 155 m²/g de catalyseur à l'état non réduit.

7. Catalyseur selon l'une ou plusieurs des revendications 1 à 6, **caractérisé en ce que** 80 à 92, avantageusement 84 à 90% de la surface spécifique totale BET sont formés par des pores d'un rayon rₚ inférieur ou égal à 15 nm.

8. Catalyseur selon une ou plusieurs des revendications 1 à 7, **caractérisé en ce que** 50 à 85, notamment 60 à 80% de la surface spécifique totale BET sont formés par des pores d'un rayon rₚ ≤ 9 nm.

9. Catalyseur selon une ou plusieurs des revendications 1 à 8, **caractérisé en ce que** 5 à 45, notamment 15 à 40, de préférence 18 à 30% de la surface spécifique totale BET sont formés par des pores d'un rayon rₚ = 9 à 15 nm.

10. Catalyseur selon une ou plusieurs des revendications 1 à 9, **caractérisé en ce qu'**il contient, pour 100 parties en poids de CuO, également 2 à 80, notamment 4 à 60, de préférence 5 à 35 parties en poids d'un support insoluble dans l'eau, notamment SiO₂ comme du kieselgur ou du gel de silice et/ou Al₂O₃, de préférence Al₂O₃.

11. Catalyseur selon une ou plusieurs des revendications 1 à 10, **caractérisé en ce qu'**il contient, sous forme non réduite, pour 100 parties en poids de Cu, 48 à 163 parties en poids de ZnO, 2,4 à 63 parties en poids de Al₂O₃ et éventuellement 0,6 à 10 parties en poids d'un oxyde de Mn, Mo, V, Zn et/ou d'un métal alcalino-terreux.

12. Catalyseur selon la revendication 11, **caractérisé en ce qu'**il contient pour 100 parties en poids de Cu, 56 à 65, notamment 56 à 100 parties en poids de ZnO, 3,7 à 50, notamment 5,0 à 37,5, de préférence de 5,0 à 13,8 parties en poids de Al₂O₃ et éventuellement 1,2 à 7,5, notamment 2,4 à 5,0 parties en poids d'un oxyde de Mn, Mo, V, Zr et/ou d'un métal alcalino-terreux.

13. Catalyseur selon la revendication 11 ou 12, **caractérisé en ce qu'**il contient de l'oxyde de Mn ou de Ba.

14. Catalyseur selon une ou plusieurs des revendications 11 à 13, **caractérisé en ce qu'**il contient, pour 100 parties en poids de Cu, 2,4 à 100, notamment 4,8 à 75, de préférence 6 à 44 parties en poids d'un support insoluble dans l'eau, notamment SiO₂, du kieselgur, du gel de silice, Al₂O₃, et de préférence Al₂O₃.

15. Procédé pour préparer un catalyseur selon une ou plusieurs des revendications 1 à 14, **caractérisé en ce qu'**on combine, en les introduisant séparément mais simultanément, une solution aqueuse contenant des sels de cuivre, de zinc, d'aluminium et éventuellement de manganèse, de molybdène, de vanadium, de zirconium et/ou d'un métal alcalino-terreux et une solution aqueuse d'un agent basique de précipitation et l'on effectue la précipitation, éventuellement en présence d'un support, à une valeur constante du pH comprise dans un intervalle allant de 7,0 à 8,5, et au-dessus de 70, en particulier de 70 à 95, de préférence de 75 à 85°C, on sépare le co-précipité obtenu, on le lave à l'eau, on le sèche, on le calcine entre 320 et 400°C en un espace de temps de 4 à 8 h et éventuellement on le réduit.

16. Procédé de préparation du catalyseur selon la revendication 15, **caractérisé en ce qu'**on soumet le catalyseur calciné à une réduction à 130 jusqu'à 190°C, éventuellement sous pression élevée, à l'aide d'un mélange azote/hydrogène qui contient 1,0 à 5% en volume d'hydrogène.

17. Utilisation du catalyseur pour l'hydrogénation d'aldéhydes en des alcools.
